# EUROPÄISCHE PATENTSCHRIFT

(11) **EP 1 150 705 B1**
(45) Veröffentlichungstag und Bekanntmachung des Hinweises auf die Patenterteilung: **26.05.2004**
(21) Anmeldenummer: 00907521.9
(22) Anmeldetag: 08.02.2000
(51) Int. Cl.: A61K 38/56, A61K 48/00, A61K 47/48, A61P 43/00, C07K 14/81, C12N 5/10

(54) **SCHUTZ VON ZELLEN MIT BBI IM ZUSAMMENHANG MIT EINER BEHANDLUNG MIT CHEMISCHEN AGENZIEN**
PROTECTION OF CELLS WITH BBI IN CONNECTION WITH TREATMENT WITH CHEMICAL AGENTS
PROTECTION DE CELLULES AVEC LE BBI DANS LE CADRE D'UN TRAITEMENT AVEC DES AGENTS CHIMIQUES

(30) Priorität: 13.02.1999 DE 19906108
(43) Veröffentlichungstag der Anmeldung: 07.11.2001
(73) Patentinhaber: Eberhard-Karls-Universität Tübingen Universitätsklinikum, 72076 Tübingen (DE)
(72) Erfinder: DITTMANN, Klaus, D-72070 Tübingen (DE); GÜVEN, Nuri, D-72766 Reutlingen (DE); MAYER, Claus, D-72072 Tübingen (DE); RODEMANN, H., Peter, D-70192 Stuttgart (DE)
(74) Vertreter: Otten, Hajo, Dr.-Ing.
(86) Internationale Anmeldenummer: PCT/EP2000/001006
(87) Internationale Veröffentlichungsnummer: WO 2000/047217

(56) Entgegenhaltungen:
- DE-A- 19 735 587
- US-A- 5 217 717
- US-A- 5 376 373
- US-A- 5 614 198
- GUEVEN NURI ET AL: "The radioprotective potential of the Bowman-Birk protease inhibitor is independent of its secondary structure." CANCER LETTERS, Bd. 125, Nr. 1-2, 13. März 1998 (1998-03-13), Seiten 77-82, XP000939303 ISSN: 0304-3835 in der Anmeldung erwähnt
- BERNKOP-SCHNURCH ANDREAS ET AL: "Intestinal peptide and protein delivery: Novel bioadhesive drug-carrier matrix shielding from enzymatic attack." JOURNAL OF PHARMACEUTICAL SCIENCES, Bd. 87, Nr. 4, April 1998 (1998-04), Seiten 430-434, XP000939327 ISSN: 0022-3549

## Beschreibung

Die vorliegende Erfindung betrifft den Schutz von Zellen im Zusammenhang mit einer in vivo- und/oder einer in vitro-Behandlung mit chemischen Agenzien.

Mit dem Schutz von Zellen im Zusammenhang mit einer in vivo- und/oder einer in vitro-Behandlung mit chemischen Agenzien wird bezweckt, die schädigende Wirkung chemischer Agenzien gegenüber Zellen in vivo und/oder in vitro zu verhindern oder zumindest zu verringern. Solche chemischen Agenzien können bspw. Zytostatika wie Cisplatin oder Fluorouracil sein, oder auch allgemein Agenzien, deren Wirkung eine Veränderung der DNA von Zellen sein kann.

Zytostatika werden bspw. in der Tumortherapie eingesetzt. Sie verhindern oder verzögern die Zellteilung von funktionell aktiven, proliferierenden Zellen durch Beeinflussung ihres Stoffwechsels. Die therapeutische Anwendung basiert darauf, daß Tumorzellen sich von normalen Körperzellen unter anderem durch eine gesteigerte Zellteilungsrate unterscheiden. Die Unterschiede zwischen normalen und Tumorzellen reichen für einen selektiven tumorspezifischen Angriffspunkt jedoch nicht aus. Daher resultieren die unerwünschten Nebenwirkungen von Zytostatika vor allem aus der generellen Regenerationhemmung rasch proliferierender Gewebe. Besonders betroffen sind die Bildung der Blutzellen, die Epitelien der Schleimhäute, deren Regenerationshemmung zu gastrointestinalen Störungen führt, und Haut und Hautanhangsgebilde, deren Regenerationshemmung zu Haarausfall führt.

Es ist bisher keine Möglichkeit bekannt, diese unerwünschten Nebenwirkungen von Zytostatika bei gleichzeitigem Erhalt ihrer therapeutischen Wirkung wirksam zu verhindern.

Es sind bereits Peptide bekannt, die Zellen vor der schädigenden Wirkung von Strahlung, insbesondere ionisierender Strahlung, schützen können, d.h. die radioprotektiv wirken; siehe bspw. die Publikation von Gueven, N., et al. (1998): "The radioprotective potential of the Bowman-Birk protease inhibitor is independent of its secondary structure", Cancer Letters 125, Seiten 77 bis 82. Auch die zytoprotektive Wirkung von Peptiden ist bereits beschrieben worden, siehe Kennedy et al. (1996): "Effects of the Bowman-Birk protease inhibitor on survival of fibroblasts and cancer cells exposed to radiation and cisplatinum", Nutr. Cancer, 26, Seiten 209-217).

Das beschriebene Peptid ist der Bowman-Birk-Protease-Inhibitor (BBI), ein seit langem bekannter Inhibitor der Serin-Proteasen Trypsin und Chymotrypsin, der in großer Menge in der Sojabohne enthalten ist. Die Aminosäuresequenz des BBI ist bekannt, das entsprechende Gen der Sojabohne wurde bereits 1984 kloniert (Hammond, R.W. (1984): "Molecular Cloning and Analysis of a Gene Coding for the Bowman-Birk Protease Inhibitor in Soybean", J.Biol.Chem. 269, Seiten 9883-9890).

Der BBI umfaßt 71 Aminosäuren und weist ein Molekulargewicht von rund 8.000 Dalton auf. Eines der Charakteristika des BBI ist die Anzahl von vierzehn Cysteinresten, die sieben Disulfidbrücken ausbilden und somit die Faltung oder Sekundärstruktur des BBI wesentlich mitbestimmen.

Durch chemische und enzymatische Spaltung mit Cyanogenbromid (CNBr) und der Protease Pepsin kann der BBI in zwei Hälften gespalten werden, von denen die eine die Trypsin-inhibierende Aktivität und die andere die Chymotrypsin-inhibierende Aktivität aufweist (Odani, S. and Ikenaka, T. (1978): "Studies on Soybean Trypsin Inhibitors", J. Biochem. 83, Seiten 747-753).

Neben der Funktion als Protease-Inhibitor wurden zwei weitere physiologische Aktivitäten des BBI nachgewiesen, nämlich eine antikarzinogene (Clair, B.H.St. (1990) "Suppression of dimethylhedrazine-induced carcinogenesis in mice by dietary addition of the Bowman-Birk protease inhibitor", Cancer Research 50, Seiten 580 bis 586) und die bereits erwähnte radioprotektive Wirkung.

Die radioprotektive Wirkung des BBI und eines BBI-Fragmentes wurde in der Publikation von Gueven et al. (1998) beschrieben. Hier konnte gezeigt werden, daß der BBI und ein Nonapeptid, das einen Teil der Aminosäure-Sequenz des BBI repräsentiert und keine Protease-inhibierende Aktivität aufweist, das durch Strahlung hervorgerufene Absterben von kultivierten Bindegewebszellen (Fibroblasten) der Haut reduziert.

In der Publikation von Kennedy et al. (1996) werden aus statistischer Sicht fragwürdige Ergebnisse präsentiert, aus denen ein moderater protektiver Effekt von BBI auf tetraploide und genetisch instabile Maus-Fibroblasten der Zelllinie 10T1/2 gegenüber der kombinierten Exposition an Cisplatin und radioaktiver Bestrahlung herausgelesen werden könnte. Eine rein zytoprotektive Wirkung von BBI, insbesondere eine solche, die selektiv gesunde Zellen schützt, ist in dieser Publikation nicht gezeigt.

Ein Problem bei der oralen Aufnahme des BBI mit der Nahrung besteht darin, daß an Ratten nachgewiesen wurde, daß große Mengen von Trypsininhibitoren zu einer Hypertrophie und Hyperplasie von Pankreaszellen sowie zu Körpergewichtsverlust führen. Bei Ratten, die für längere Zeiträume Trypsininhibitoren aus Sojabohnen mit der Nahrung aufnahmen, entwickelten sich sogar Pankreastumore.

Ein weiteres Problem bei der Verabreichung von Serin-Protease-Inhibitoren, vor allem bei einer intravenösen Verabreichung, besteht darin, daß die Blutgerinnung, an der Serin-Proteasen maßgeblich beteiligt sind, gestört werden kann.

Vor diesem Hintergrund ist es Aufgabe der vorliegenden Erfindung, ein Mittel zum Schutz von Zellen im Zusammenhang mit einer in vivo- und/oder einer in vitro-Behandlung mit chemischen Agenzien bereitzustellen.

Diese Aufgabe wird erfindungsgemäß dadurch gelöst, daß zur Herstellung eines entsprechenden Mittels ein Peptid verwendet wird, das ein Fragment des BBI umfaßt.

Die Erfinder der vorliegenden Anmeldung haben überraschenderweise festgestellt, daß auch modifizierte Formen von BBI, nicht nur eine radioprotektive Aktivität aufweisen, sondern auch einen Schutz von Zellen vor chemischen Agenzien bieten. Noch überraschender war, daß die ggf. modifizierten Fragmente des BEI Zellen vor der Wirkung chemischer Agenzien schützen können, wobei diese Fragmente nicht einmal inhibierend auf Trypsin oder Chymotrypsin wirken müssen.

Dabei wird unter modifiziert jede Änderung der Struktur bzw. Konformation des BBI sowie jede Abwandlung seiner Aminosäureabfolge verstanden, sei es durch chemisches oder enzymatisches Hinzufügen oder Wegnehmen einzelner Gruppen von Aminosäuren oder durch Austausch einzelner Aminosäuren. Eine modifizierte Form des BBI beinhaltet auch ein Peptid, bei dem weitere Aminosäuren an das N- oder C-terminale Ende des BBI angehängt wurden, bspw. Domänen weiterer Proteine oder Peptide, die die Reinigung des BBI erleichtern und/oder dessen Zellen schützende Wirkung noch verstärken.

Unter einem Fragment im Sinne der Erfindung wird jedes Teilstück des BBI verstanden, bei dem entweder nur einzelne Aminosäuren oder größere Aminosäureabschnitte des BBI fehlen. Derartige Fragmente umfassen bspw. einzelne Domänen des BBI. Erfindungsgemäß können solche Fragmente auch modifiziert im oben dargelegten Sinne sein.

Modifizierte Formen der BBI-Fragmente können entweder durch Behandlung des nativen, also unveränderten BBI mit Chemikalien oder Enzymen oder durch Synthese mit chemischen oder molekularbiologischen Methoden erzeugt werden.

Daß auch modifizierte Fragemente des BBI eine Zellen schützende Wirkung im Sinne der Erfindung aufweisen können, war nicht zu erwarten, da üblicherweise Modifikationen, vor allem wenn sie die Konformation des Peptids betreffen, die physiologischen Aktivitäten eines Peptids zerstören oder zumindest stark reduzieren.

Darüber hinaus war für die antikarzinogene Aktivität des BBI gezeigt worden, daß durch Beeinträchtigung der BBI-Struktur durch Hitzedenaturierung dessen antikarzinogene Wirkung völlig verloren ging.

Unter einer in vivo-Behandlung mit chemischen Agenzien sind Chemotherapien zu verstehen, wie sie bspw. im Rahmen einer Tumortherapie, einer Immunsuppression oder einer anti-viralen Therapie durchgeführt werden.

Unter einer in vitro-Behandlung mit chemischen Agenzien versteht man, daß isolierte Zellen oder Gewebe außerhalb eines Körpers chemischen Agenzien ausgesetzt werden. Dies kann bspw. im Rahmen einer Leukämie-Therapie erfolgen, bei der dem Patienten Knochenmarkzellen entnommen werden, um sie nach Behandlung mit chemischen Agenzien wieder in den Patienten einzusetzen.

Auch eine Behandlung von entnommenen Organen, die für eine Transplantation vorbereitet werden, ist denkbar.

Unter dem Schutz von Zellen ist zu verstehen, daß die eingesetzten chemischen Agenzien bei bestimmten Zellen nicht oder nur eingeschränkt wirken.

Unter der Verwendung eines Peptides ist zu verstehen, daß dieses Peptid entweder in einer geeigneten galenischen Aufarbeitung in einen Organismus eingebracht wird oder das Peptid in vitro in irgendeiner Form mit isolierten Zellen oder Geweben in Kontakt gebracht wird. Dabei kann die Applikation des Peptides vor, während oder nach der Behandlung mit chemischen Agenzien erfolgen.

Somit wird die der Erfindung zugrundeliegende Aufgabe vollkommen gelöst.

In einer bevorzugten Ausgestaltung weist das verwendete Peptid keine oder zumindest keine wesentliche Protease-inhibierende Wirkung gegen Trypsin und Chymotrypsin auf.

Ein derartiges Peptid hat den erheblichen Vorteil, neben der Zellen schützenden Wirkung nicht gleichzeitig zusätzlich noch die Proteasen Trypsin oder Chymotrypsin zu blockieren. Dies ist besonders dann vorteilhaft, wenn das Peptid zum Schutz von Zellen, Geweben oder Organismen gegen die Wirkung chemischer Agenzien eingesetzt werden soll, denn, wie oben bereits erwähnt wurde, sind zumindest Peptide mit Trypsin-inhibierender Wirkung schädlich für Pankreaszellen und können sogar zur Tumorbildung führen.

Die Tatsache, daß modifizierte Formen des BBI Zellen gegen die Wirkung chemischer Agenzien schützen können, ohne zugleich eine wesentliche Protease-inhibierenden Aktivität gegen Trypsin oder Chymotrypsin zu haben, war umso erstaunlicher, als bisher davon ausgegangen wurde, daß für die antikarzinogene Wirkung des BBI eine dieser Protease-inhibierenden Domänen notwendig sei (Clair et al., 1990).

In einer weiteren bevorzugten Ausgestaltung weist das verwendete Peptid zumindest zwei Cysteinreste auf, die in reduzierter Form vorliegen.

Ein Peptid, dessen Cysteinreste in reduzierter Form vorliegen, hat den Vorteil, daß das Peptid problemlos mit molekularbiologischen Methoden in Bakterien hergestellt werden kann. Eine solche Expression in Bakterien ist gegenüber der Expression in Hefen und höheren eukaryontischen Zellen wesentlich einfacher und effizienter und erlaubt besonders hohe Ausbeuten. Für Proteine oder Peptide, bei denen die Cysteinreste über Disulfidbrücken kovalent miteinander verbunden sind, ist dieses System jedoch ungeeignet, da in Bakterien keine Disulfidbrücken ausgebildet werden können. Eine reduzierte Form des BBI kann dagegen ohne weiteres in Bakterien exprimiert werden.

In einer weiteren bevorzugten Ausgestaltung weist das verwendete Peptid weniger als 20, vorzugsweise weniger als zehn Aminosäuren auf.

Ein solches drastisch verkleinertes BBI-Fragment hat den erheblichen Vorteil, sich bei seinem Einsatz auf Zellen, Geweben oder im ganzen Organismus aufgrund der geringen Größe wesentlich besser und schneller zu verteilen und einzudringen als der über 70 Aminosäuren umfassende Gesamt-BBI.

Eine gute Verteilung sowie ein leichtes Eindringen in das Gewebe ist bei der Verwendung des Peptides zum Schutz von Zellen wesentlich, da es dann schnell und umfassend seine schützende Wirkung gegenüber chemischen Agenzien entfalten kann.

In einer weiter bevorzugten Ausführung ist das verwendete Peptid ein Nonapeptid und weist die Sequenz SEQ ID-Nr.: 1 aus dem anliegenden Sequenzprotokoll auf. Es kann auch eine dazu homologe Sequenz aufweisen.

Wie den Ausführungsbeispielen zu entnehmen ist, ist es den Erfindern gelungen, nachzuweisen, daß dieses Nonapeptid ebenso wie das Gesamt-BBI eine schützende Wirkung auf menschlichen Bindegewebszellen der Haut (Hautfibroblasten) gegenüber chemischen Agenzien, wie bspw. Cisplatin, ausübt. Der Einsatz eines gegenüber dem Gesamt-BBI sehr stark verkleinerten Peptids, das nur neun Aminosäuren umfaßt (Nonapeptid), hat den wesentlichen Vorteil, daß dieses Nonapeptid leichter hergestellt werden kann. Ein Nonapeptid kann nämlich problemlos durch chemische Synthese, auch Merrifield-Synthese genannt, produziert werden. Diese Art der Peptidsynthese ist eine weit verbreitete und gut etablierte Synthesemethode, durch die ein derartiges Peptid in großen Mengen und in hoher Reinheit gewonnen werden kann.

In einer weiteren bevorzugten Ausführung ist das verwendete Peptid ein Nonapeptid mit der Sequenz SEQ ID-Nr.: 2 aus dem anliegenden Sequenzprotokoll.

Bei diesem Nonapeptid wurde die aus dem natürlich vorkommenden BBI entnommene Sequenz um eine Aminosäure abgewandelt. Das Serin aus der Sequenz SEQ ID-Nr.: 1, also eine hydrophile Aminosäure, wurde in der SEQ ID-Nr.: 2 gegen ein Valin, also eine hydrophobe Aminosäure, ausgetauscht. Auch dieses modifizierte Nonapeptid mit der Sequenz SEQ ID-Nr.: 2 kann problemlos mittels Merrifield-Synthese synthetisiert werden.

In einer weiteren bevorzugten Ausführung weist das verwendete Peptid die Sequenz SEQ ID-Nr.: 3 aus dem anliegenden Sequenzprotokoll auf.

Dieses Peptid umfaßt nur sieben Aminosäuren, es ist also ein Heptapeptid. Durch die weitere Verkürzung des Peptids unter Erhalt seiner Zellen schützenden Wirkung ist das Peptid noch schneller und preiswerter herzustellen sowie leichter zu applizieren.

In einer weiteren bevorzugten Ausgestaltung liegt zumindest einer der Aminosäurereste des verwendeten Peptides in alkylierter Form vor.

Unter Alkylierung versteht man die Modifikation einzelner Aminosäuren mit Alkylgruppen, meist Methylgruppen. Diese Modifikation erfolgt mit sogenannten Alkylierungsreagenzien, bspw. mit Jodacetamid.

Durch die Behandlung des BEI mit Jodacetamid nach Reduktion der Disulfidbrücken werden die SH-Gruppen der Cysteine mit einer Methylgruppe versehen, also alkyliert. Eine Reoxidation der SH-Gruppen ist dann nicht mehr möglich. So wird unter oxidierenden Bedingungen verhindert, daß sich Disulfidbrücken ausbilden, die zu einer Fehlfaltung eventuell unter Polymerbildung führen, so daß die Zellen schützende Wirkung möglicherweise verloren geht.

Eine Alkylierung des BBI oder von Fragmenten des BBI hat den Vorteil, die Cysteinreste in ihrer reduzierten Form zu erhalten und damit zu stabilisieren.

In einer weiteren bevorzugten Ausführung sind die terminalen Cysteinreste des verwendeten Peptides kovalent miteinander verbunden.

Auf diese Weise entstehen ringförmige oder zyklische Peptide, die in einem oxidierenden Milieu eine hohe Stabilität aufweisen. Ein oxidierendes Milieu liegt bspw. in der extrazellulären Matrix des Bindegewebes sowie an allen Körperteilen vor, die Kontakt mit der Außenluft haben.

Alle Peptide mit den Sequenzen SEQ ID-Nr.: 1 - 3, seien sie ringförmig oder linear, haben den erheblichen Vorteil, keine wesentliche Protease-inhibierende Aktivität zu haben. Sie können somit ohne die unerwünschte Nebenwirkung der Blockierung der Verdauungsenzyme Trypsin und Chymotrypsin und anderer Serin-Proteasen eingesetzt werden.

In einer weiteren bevorzugten Ausgestaltung weist zumindest eine der Aminosäuren des verwendeten Peptides eine Schutzgruppe auf.

Derartige Schutzgruppen können beliebige der in der Peptidchemie bekannten Schutzgruppen sein, wobei es bevorzugt ist, daß die N-terminale Aminosäure eine Acetylgruppe und/oder die C-terminale Aminosäure eine Amidgruppe aufweist.

Diese Schutzgruppen haben den Vorteil, die Peptide vor dem Angriff von Exopeptidasen zu schützen, so daß die Peptide in einem biologischen Milieu wie bspw. in der Zellkultur oder im Organismus eine wesentlich höhere Stabilität haben. Schutzgruppen, die die C-terminale Carboxyl- und die N-terminale Aminogruppe von Peptiden blockieren, wie die genannten Amid- bzw. Acetylgruppen, sorgen ferner dafür, daß die Peptide keine weiteren Peptidbindungen mit anderen Peptiden oder untereinander eingehen, so daß auch Polymerisierungen der Peptide sicher verhindert werden. Bei mehreren untereinander verknüpften Peptiden kann nicht mehr sicher davon ausgegangen werden, daß die Zellen schützende Wirkung erhalten ist.

Demnach sorgen die Schutzgruppen auch dafür, daß die Peptide in ihrer wirksamen Struktur erhalten bleiben.

Die Erfindung betrifft ferner die Verwendung zumindest einer Nukleinsäure zur Herstellung eines Mittels zum Schutz von Zellen im Zusammenhang mit einer Behandlung mit chemischen Agenzien. Diese Nukleinsäure umfaßt einen Sequenzabschnitt, der für mindestens eines der vorgenannten Peptide codiert. Darüber hinaus weist die verwendete Nukleinsäure ggf. weitere Sequenzen auf, die zur Expression der dem Peptid entsprechenden Nukleinsäuresequenz notwendig sind. Die verwendete Nukleinsäure kann in einem Vektor enthalten sein, der geeignet ist, die Expression der dem Peptid entsprechenden Nukleinsäuresequenz in einer Zelle zu ermöglichen.

Eine derartige Nukleinsäure hat den Vorteil, daß sie chemisch stabiler und unempfindlicher ist als Peptide. Nukleinsäuren können unter den üblichen Bedingungen nämlich kaum denaturieren und nicht wie Proteine Konformationsänderungen erfahren, die sie funktionsunfähig machen. Die Handhabung ist daher einfacher als die der Peptide und die Lagerfähigkeit von Nukleinsäuren ist nahezu unendlich. Sie sind chemisch und/oder molekularbiologisch sehr günstig und im Prinzip in unbegrenzten Mengen herzustellen. Eventuell zur Expression notwendige Nukleinsäuresequenzen und ein Vektor, der die Nukleinsäure enthält, haben den Vorteil, es den behandelten Zellen zu erlauben, effektiv Peptide herzustellen, die Zellen vor der Wirkung chemischer Agenzien schützen.

Ein weiterer Aspekt der Erfindung betrifft die Verwendung einer solchen Nukleinsäure im Rahmen einer Gentherapie. Dabei wird die Nukleinsäure dem Patienten in einer Form verabreicht, die es der Nukleinsäure ermöglicht, von Zellen aufgenommen zu werden.

Der Vorteil dieser Verwendung besteht darin, daß körpereigene Zellen die Erzeugung der Peptide übernehmen, so daß diese Peptide, zumindest in dem Zeitraum, der im Zusammenhang mit einer Behandlung mit chemischen Agenzien relevant ist, in ausreichender Menge im Körper zur Verfügung stehen und nicht von außen zugeführt und dosiert werden müssen.

Die Erfindung betrifft darüber hinaus die Verwendung einer der beschriebenen Nukleinsäuren zur Transformation oder Transfektion von Zellen in vitro.

Der Einsatz der Nukleinsäure in vitro hat den Vorteil, daß Verfahren, wie bspw. die Elektroporation, und/oder Hilfsstoffe, wie bspw. Calciumphosphat oder DEAE-Dextran, verwendet werden können, die die Aufnahme von Nukleinsäuren in Zellen wesentlich erleichtern und verbessern, die aber in vivo nicht einsetzbar sind. Dabei können beispielsweise Zellen behandelt werden, die aus einem Patienten entnommen wurden und später wieder in diesen Patienten eingebracht werden.

Ein weiterer Aspekt der vorliegenden Erfindung ist eine der genannten Verwendungen zum Schutz von Zellen im Zusammenhang mit einer Behandlung mit
- Zytostatika, insbesondere Alkylanzien, wie Cisplatin oder Nitroso-Harnstoff-/-Guanidin-Verbindungen, Antimetabolite, wie Fluorouracil und/oder Antibiotika, wie Bleomycin;
- Virostatika, insbesondere Nukleosidanaloga, wie Azidothymidin, Aciclovir oder Idoxuridin; und/oder
- immunsupprimierenden Mitteln, wie bspw. Azathioprin.

Die Nachteile derartiger bekannter Behandlungen bestehen in den erheblichen unerwünschten Nebenwirkungen, wie bspw. einer generellen Regenerationshemmung rasch proliferierender Gewebe, insbesondere bei Blutzellen, Schleimhautepitelien, Haut und Hautanhangsgebilden. Darüber hinaus bewirken viele dieser Agenzien eine unerwünschte Immunsuppression und sind zum Teil potentiell karzinogen. Der Vorteil der Verwendung der Peptide und/oder Nukleinsäuren zum Schutz von Zellen besteht darin, daß diese Nebenwirkungen bei erhaltener Therapiewirkung verringert oder verhindert werden können. Die Erfinder konnten nämlich zeigen, daß der Zellen schützende Effekt bei Zytostatika-Behandlung nicht bei transformierten, wohl aber bei normalen Hautfibroblasten eintritt. Darüber hinaus ist es denkbar, daß es von der Konzentration der Peptide abhängt, ob bestimmte Zellen des Körpers geschützt werden oder nicht. So können bei einer Immunsuppression bspw. die Schleimhautepithelien gegenüber den chemischen Agenzien geschützt sein, während die Zellen des Immunsystems nicht geschützt sind. Schleimhautepithelien können auch dadurch geschützt werden, daß die Anwendung der Peptide und/oder Nukleinsäuren von der epithelialen Seite her erfolgt, während die chemischen Agenzien systemisch eingesetzt werden.

Ein weiterer, mit der Verringerung oder Verhinderung der Nebenwirkungen einhergehender Vorteil besteht darin, daß die Zytostatika bzw. Virostatika höher dosiert eingesetzt werden können, weil die Höhe der eingesetzten Dosis normalerweise durch die unerwünschten Nebenwirkungen limitiert wird. Somit kann ein besserer Therapieerfolg ermöglicht werden.

Besonders bevorzugt ist die Kopplung oder Bindung der erfindungsgemäß verwendeten Peptide und/oder Nukleinsäuren an einen Hilfsstoff, wie bspw. ein Protein oder eine lipophile, amphiphile oder hydrophile Verbindung.

Diese Kopplung kann die Aufnahme der Peptide oder der Nukleinsäuren in den Körper und/oder in Zellen ermöglichen oder auch verhindern, je nachdem, welcher Hilfsstoff gewählt und welcher Effekt gewünscht wird. So kann bspw. ein Peptid, das im Gastrointestinaltrakt bleiben soll, an eine Verbindung gekoppelt sein, die nicht im Darm aufgenommen wird.

Darüber hinaus besteht durch die geringe Größe der Peptide und Nukleinsäuren das Problem, daß diese sehr schnell über die Niere wieder ausgeschieden werden, wenn sie sich im Blut befinden. Deshalb ist zur Verlängerung der Halbwertzeit im Blut eine Kopplung an größere Moleküle unter bestimmten Voraussetzungen vorteilhaft.

Ein weiterer Aspekt der vorliegenden Erfindung betrifft die Verwendung des in Rede stehenden Peptids oder der in Rede stehenden Nukleinsäure in einer Zellen gegenüber Zytostatika, immunsupprimierenden Mitteln und/oder Virostatika schützenden wirksamen Menge als Bestandteil einer pharmazeutischen Zusammensetzung.

Dazu können die Peptide und/oder Nukleinsäuren in den jeweils angemessenen und üblichen Galeniken zubereitet sein, die bspw. auch eine Zubereitung mit Liposomen oder Vesikeln umfassen. Neben einer intravenösen Verabreichung kann auch ein lokales Einbringen, bspw. durch eine Injektion in betroffene Körpergebiete oder Körperhöhlen, eine oberflächliche Anwendung, die auch innere Körperoberflächen einbezieht, eine orale Verabreichung oder eine Verabreichung in Form von Suppositorien in Betracht gezogen werden. Für Nukleinsäuren ist es vorteilhaft, im Inneren von Vesikeln oder Liposomen vor einem Abbau durch Nukleasen geschützt zu sein. Gleichzeitig ermöglichen Liposomen gut das Eindringen der Nukleinsäuren in das Innere der Zellen. Weiterhin ist bei der pharmazeutischen Zusammensetzung vorteilhaft, daß sie durch das Verwenden von Hilfsstoffen eine möglichst schnelle und effiziente Wirkung der verwendeten Peptide und/oder der Nukleinsäuren ermöglicht.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung werden in der nachfolgenden Beschreibung näher erläutert und sind in der Zeichnung dargestellt, in der
- Fig. 1: das Ergebnis eines sogenannten klonogenen Assays mit und ohne BBI bei Behandlung normaler Hautfibroblasten mit Cisplatin in Form eines Kurvendiagrammes zeigt;
- Fig. 2: als Balkendiagramm das Ergebnis eines klonogenen Assays mit und ohne dem SEQ ID-Nr.: 1 entsprechenden Peptid P1 bei Behandlung normaler Hautfibroblasten mit Cisplatin zeigt;
- Fig. 3: als Balkendiagramm das Ergebnis eines klonogenen Assays mit und ohne dem SEQ ID-Nr.: 1 entsprechenden Peptid P1 bei Behandlung transformierter Hautfibroblasten mit Cisplatin zeigt;
- Fig. 4: als Balkendiagramm die Chymotrypsin-inhibierende Aktivität von BBI und vier erfindungsgemäßen Peptiden zeigt; und
- Fig. 5: als Balkendiagramm die Trypsin-inhibierende Aktivität von BBI und vier erfindungsgemäßen Peptiden zeigt.

### Beispiel 1: Zellen schützende Wirkung von BBI bei Behandlung normaler Hautfibroblasten mit Cisplatin

Die Untersuchung der Zellen schützenden Wirkung des BBI sowie modifizierter Formen des BBI wurde im sogenannten klonogenen Assay durchgeführt, der bspw. bei Dittmann, K. et al., Radiotherapy and Oncology 34 (1995), Seiten 137 bis 143, beschrieben ist und der im folgenden kurz erläutert wird.

### 1.1 Klonogener Assay (= Klonbildungsassay)

Normale humane Hautfibroblasten, die aus Biopsiematerial (Vorhaut) von einem 1,5 Jahre alten männlichen Spender etabliert wurden, und HH4DD-Zellen (in vitro spontan transformierte menschliche Hautfibroblasten, die eine Punktmutation im Gen für p53 aufweisen, die zu einem nicht funktionellen p53-Protein führen) wurden in Dulbecco's Modified Eagles Medium (DMEM) mit 10 % fötalem Kälberserum (Normalmedium) unter Standardbedingungen kultiviert. Bei jeder Zellpassage wurden die Zellen beim Anlegen von Subkulturen mit einer Dichte von 2 x 10⁴ Zellen pro cm² ausgesät.

Für den klonogenen Assay wurden die Zellen bis zur Konfluenz kultiviert. Anschließend wurde das Medium gewechselt und die Zellen entweder mit Normalmedium oder mit Medium, das 10 µM BBI enthielt, 16 Stunden lang inkubiert. Unmittelbar nach dieser Vorbehandlung wurden die Zellen mit 1 µg/ml Cisplatin (cis-Diamminplatin(II)-dichlorid) eine Stunde lang behandelt. Nach der Behandlung wurde das Medium entfernt. Die Zellen wurden gewaschen und unter Standardbedingungen bis zu den in den Graphiken angegebenen Zeiten, also 0, 1, 3, 6 und zum Teil 24 Stunden in Normalmedium inkubiert. Anschließend wurden die Zellen mit 0,05 % Trypsin und 0,1 % EDTA abgelöst, zur Analyse der Kolonie-Bildung in einer Dichte von 500 Zellen pro 78 cm²-Schale ausgesät und in Kulturmedium mit 20 % fötalem Kälberserum inkubiert.

Danach wurden die Zellen 14 Tage weiter kultiviert, um eine Kolonie-Bildung zu erlauben. Dann wurden die Zellen fixiert, gefärbt und ausgezählt.

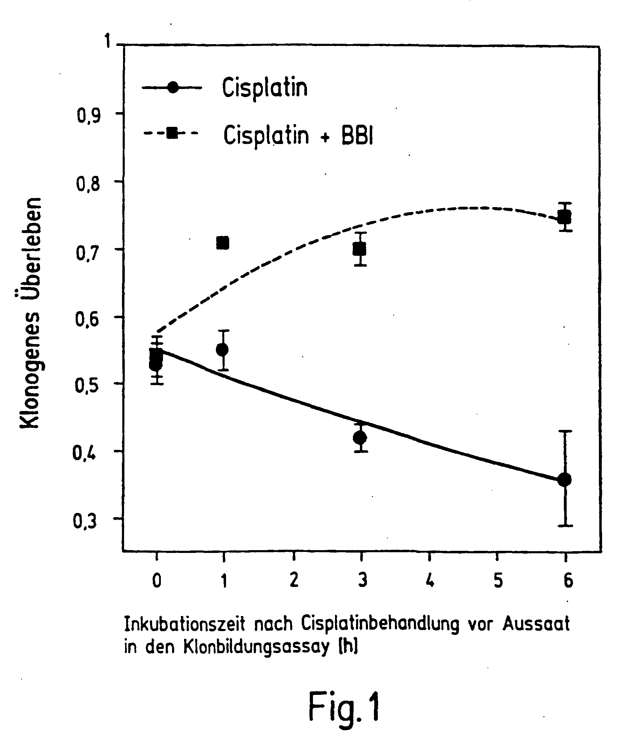

In den Fig. 1, 2 und 3 ist der Anteil der Zahl gebildeter Kolonien an der Zahl gebildeter Kolonien einer unbehandelten Kontrolle (klonogenes Überleben) in Abhängigkeit von der Inkubationszeit zwischen Cisplatinbehandlung und Ablösen der Zellen aufgetragen.

Ein Klon ist dabei eine Kolonie oder ein Zellhaufen, der durch Teilung einer Zelle innerhalb der 14-tägigen Kultivierung entstanden ist. Die Anzahl an Klonen entspricht dem Ausmaß, in dem die Zellen die Behandlung überlebt haben. Es wird daher im folgenden auch von dem "klonogenen Überleben" der Zellen gesprochen. Wenn bei einer Behandlung der Zellen viele Zellen sterben, so bilden sich nach 14 Tagen nur wenige Klone aus, überleben viele Zellen, so lassen sich nach 14-tägiger Kultivierung viele Klone auszählen. Somit ist das klonogene Überleben der Zellen nach einer Behandlung ein direktes Maß für die Zellen schützende Wirkung des BBI bzw. modifizierter Formen des BBI.

### 1.2 Zellen schützende Wirkung von BBI (Referenz Beispiel)

Bei dem klonogenen Assay, dessen Ergebnis in Fig. 1 gezeigt ist, wurden normale humane Hautfibroblasten vor ihrer einstündigen Behandlung mit Cisplatin 16 Stunden lang mit BBI vorbehandelt. Bei einem Kontrollansatz erfolgte diese Vorbehandlung nicht.

Dieser Test wurde in mehreren unabhängigen Experimenten wiederholt und die jeweiligen Ergebnisse gemittelt. Die Fehlerbalken geben die Standardabweichung der Werte an.

Wie Fig. 1 zu entnehmen ist, verringert die Behandlung konfluenter Fibroblasten mit Cisplatin das klonogene Überleben von sofort ausgesäten Zellen gegenüber unbehandelten Kontrollzellen um etwa 50 %. Sogar nach Entfernen von Cisplatin aus dem Kulturmedium zeigen die Zellpopulationen, die bis zu 6 Stunden nach der Behandlung inkubiert werden, eine zeitabhängige weitere Verringerung des klonogenen Überlebens. Jedoch führt die Vorbehandlung mit BBI zu einer signifikanten Erhöhung des klonogenen Überlebens, die schon bei einer Stunde Inkubation nach der Behandlung beobachtet werden kann.

Das in Fig. 1 gezeigte Ergebnis des klonogenen Assays belegt, daß BBI normale Hautfibrolasten effektiv vor der Wirkung von Cisplatin schützt.

### Beispiel 2: Zellen schützende Wirkung von dem der Sequenz SEQ ID-Nr.: 1 entsprechenden Peptid P1 bei normalen Hautfibroblasten

### 2.1 Klonogener Assay

Das der Sequenz SEQ ID-Nr.: 1 entsprechende Peptid P1 wurde in einem klonogenen Assay eingesetzt, wie er unter 1.1 beschrieben wurde. Dabei wurden die Fibroblasten vor der Behandlung mit Cisplatin 16 Stunden lang mit dem Peptid P1 in einer Konzentration von 80 µM behandelt.

### 2.2 Zellen schützende Wirkung des SEQ ID-Nr.: 1 entsprechenden Peptides

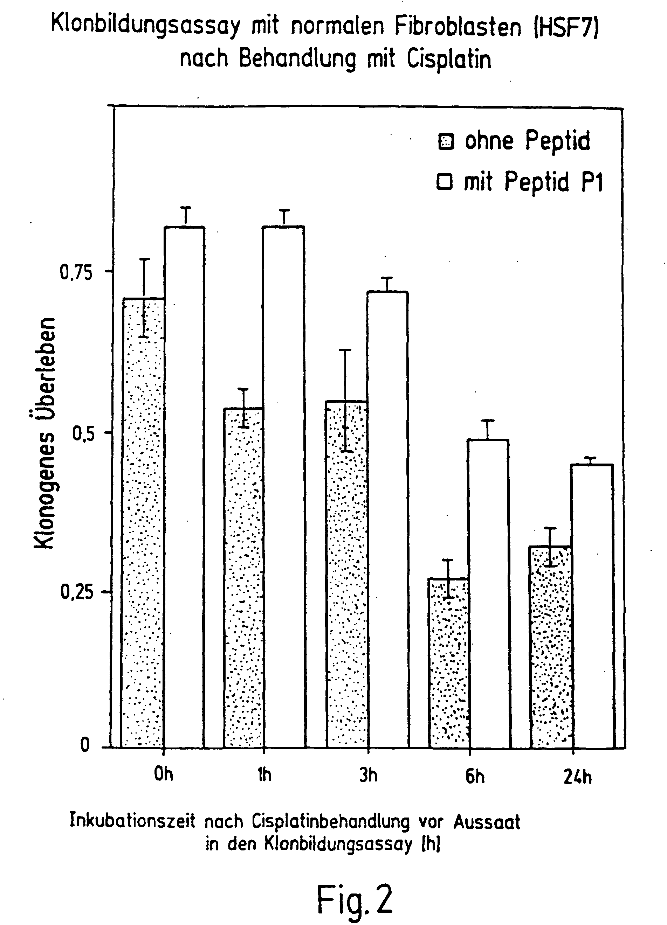

Die Vorbehandlung der normalen Hautfibroblasten mit dem der Sequenz SEQ ID-Nr.: 1 entsprechenden Peptid P1 führt gemäß Fig. 2 bereits bei einer einstündigen Inkubation nach Cisplatinbehandlung zu einer deutlichen Steigerung (ca. 50 %) des klonogenen Überlebens gegenüber Hautfibroblasten, die nicht mit dem Peptid P1 vorbehandelt wurden. Das klonogene Überleben nicht vorbehandelter und vorbehandelter Hautfibroblasten verringert sich bis zu einer Inkubationszeit von 6 h nach Cisplatinbehandlung und bleibt dann innerhalb der Fehlerbereiche bis zu einer Inkubationszeit von 24 h nach Cisplatinbehandlung etwa konstant. Nach 6 h Inkubationszeit übersteigt das klonogene Überleben der mit Peptid P1 vorbehandelten Hautfibroblasten das klonogene Überleben nicht vorbehandelter Hautfibroblasten um etwa 80 %.

Dies zeigt, daß das der Sequenz SEQ ID-Nr.: 1 entsprechende Peptid P1 wie das Gesamt-BBI normale Hautfibroblasten vor der Wirkung von Cisplatin schützt.

### Beispiel 3: Zellen schützende Wirkung von dem der Sequenz SEQ ID-Nr.: 1 entsprechenden Peptid P1 bei transformierten Hautfibroblasten

### 3.1 Klonogener Assay

Die Durchführung des Assays erfolgte wie unter 2.1 beschrieben.

### 3.2 Zellen schützende Wirkung des SEQ ID-Nr.: 1 entsprechenden Peptides P1

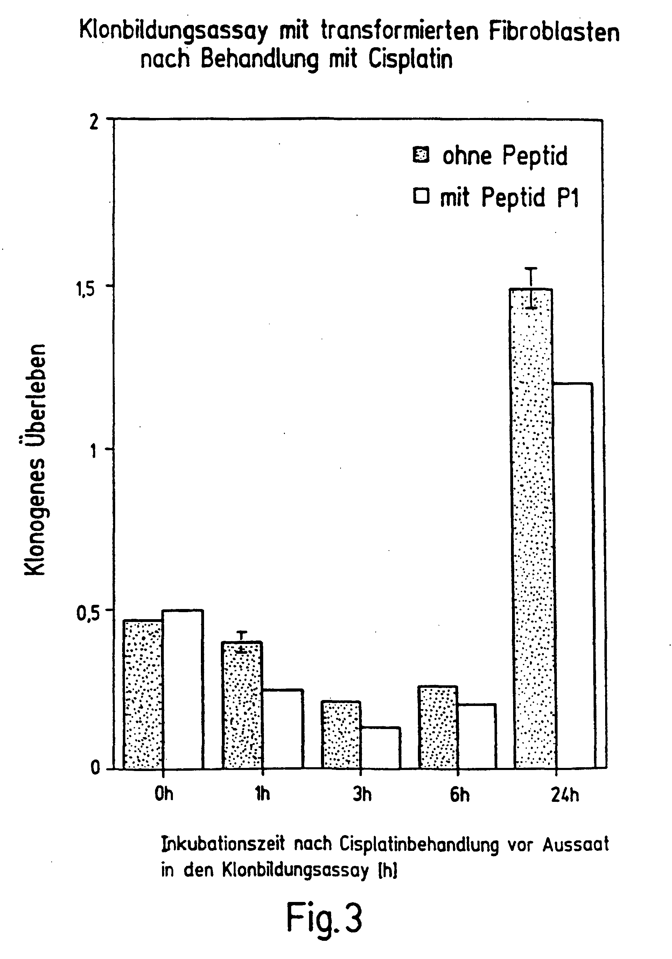

Wie Fig. 3 zeigt, ist das klonogene Überleben vorbehandelter und nicht vorbehandelter HH4DD-Zellen bis zu einer Inkubationszeit von 6 h nach Cisplatinbehandlung verringert und steigt bis zu einer Inkubationszeit von 24 h infolge der entarteten Wachstungskontrolle dieser Zellen stark an. Abgesehen von den Zellen, die sofort nach der Behandlung ausgesät wurden und bei denen das klonogene Überleben vorbehandelter und nicht vorbehandelter Zellen etwa gleich ist, führt das der Sequenz SEQ ID-Nr.: 1 entsprechende Peptid P1 bei der transformierten Fibroblastenzellinie HH4DD zu keinem gesteigerten klonogenen Überleben. Im Gegenteil, die Vorbehandlung mit dem Peptid P1 führt bei den transformierten Fibroblasten sogar zu einem weiter reduzierten klonogenen Überleben.

Dieses Ergebnis belegt, daß das der Sequenz SEQ-ID-Nr.: 1 entsprechende Peptid P1 nicht in der Lage ist, transformierte Zellen vor der Wirkung von Cisplatin zu schützen.

Die Ergebnisse zeigen, daß das erfindungsgemäß verwendete Peptid, das den ggf. modifizierten BBI aufweist, zum Schutz von Zellen gegenüber chemischen Agenzien geeignet ist und daß sich dieser Schutz auf normale, nicht aber auf entartete Zellen erstreckt.

Dies ist besonders vorteilhaft, weil man im Falle einer Tumortherapie mit Zytostatika natürlich nur die gesunden Körperzellen, nicht aber die entarteten Zellen des Tumors, vor der Wirkung der Zytostatika schützen möchte.

### Beispiel 4 Protease-inhibierende Aktivität der Peptide

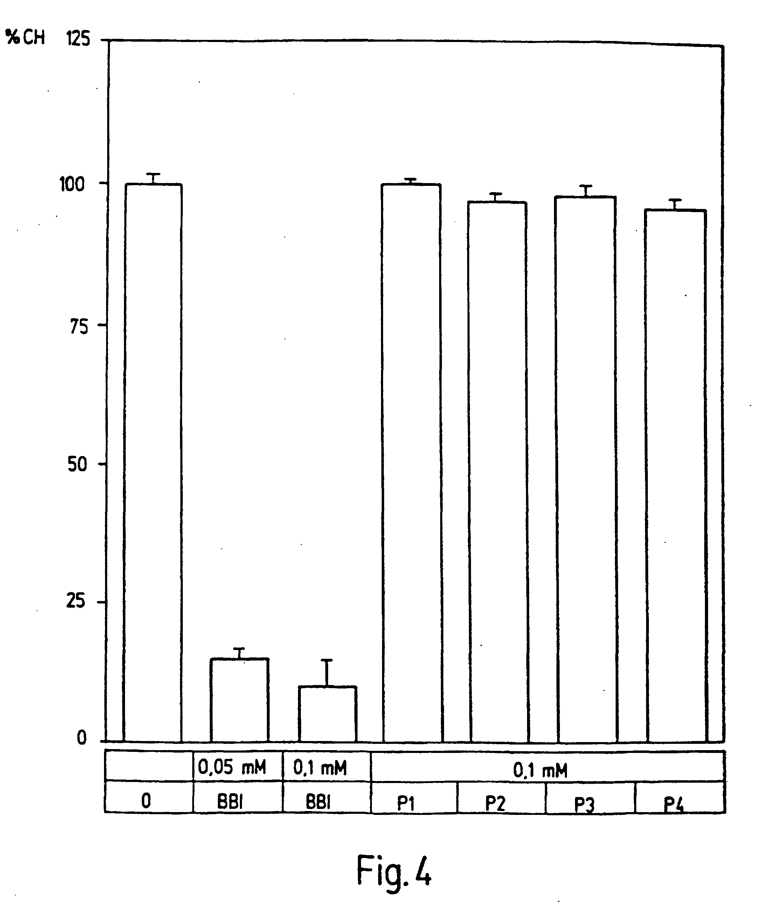

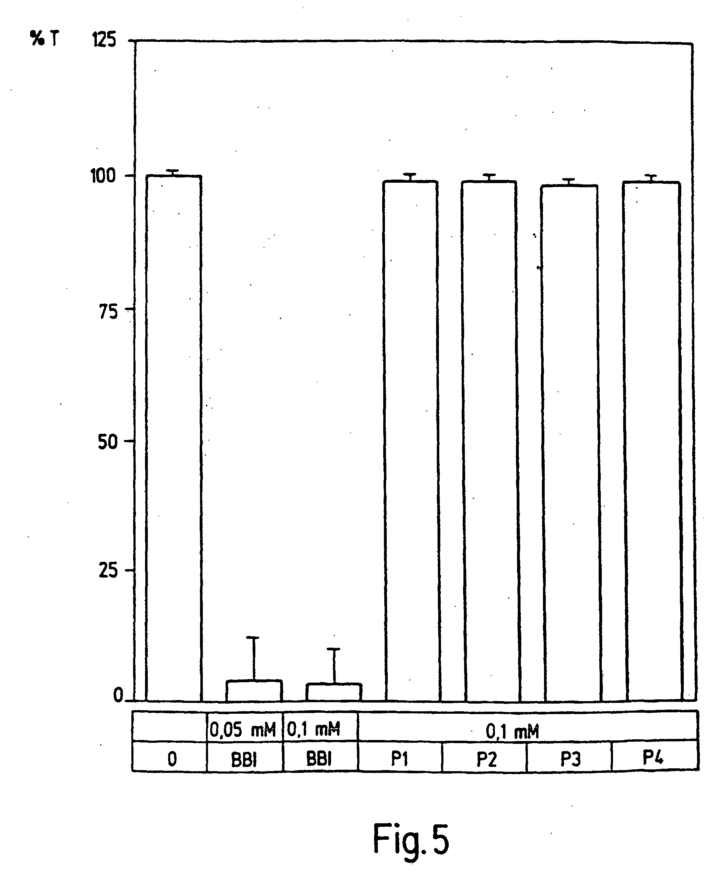

In den Experimenten, deren Ergebnisse in den Fig. 4 und 5 gezeigt sind, wurde die Protease-inhibierende Aktivität des BBI mit derjenigen der Peptidfragmente P1 bis P4 verglichen.

Das Peptid P1 weist die Sequenz SEQ ID-Nr.: 1 auf, wobei seine terminalen Cysteinreste über eine Disulfidbrücke vernetzt sind, so daß das Peptid P1 eine Ringstruktur aufweist. Das Peptid P2 hat die Sequenz SEQ ID-Nr.: 3. Es enthält keine Cysteinreste und nimmt daher eine lineare Konformation ein.

Das Peptid P3 entspricht dem Peptid P1, hat also die Sequenz SEQ ID-Nr.: 1, wobei seine terminalen Cysteinreste jedoch nicht über eine Disulfidbrücke vernetzt sind.

Das Peptid P3 hat somit wie P2 eine lineare Struktur.

Das Peptid P4 hat die Sequenz SEQ ID-Nr.: 2 und weist damit gegenüber den Peptiden P1 und P3, die die Sequenz SEQ ID-Nr.: 1 haben, einen Serin → Valin Aminosäureaustausch auf. Seine terminalen Cysteinreste sind nicht über eine Disulfidbrücke vernetzt, so daß auch das Peptid P4 eine lineare Struktur aufweist.

Die Peptide P1, P3 und P4 sind Nonapeptide mit neun Aminosäuren, das Peptid P2 ist dagegen ein Heptapeptid mit nur sieben Aminosäuren.

Der Test der Protease-Inhibition wurde wie folgt durchgeführt:

Ein Ansatz mit 50 µl TLCK-behandeltem Chymotrypsin (0,1 mg/ml) wurde mit 50 µl einer Lösung mit 0,05 mM BBI, 0,1 mM BBI oder 0,1 mM Peptid P 1, 2, 3 oder 4 zusammen mit 50 µl PBS und 50 µl des Chymotrypsin-Substrates Acetyl-A-A-P-F-pNa (0,5 mg/ml von Bachem Heidelberg, BRD) in 12,5 % DMSO, 87,5 % PBS für 10 Minuten inkubiert. Das farbige Reaktionsprodukt wird bei 405 nm in einem Spektrophotometer nachgewiesen.

Zur Bestimmung der Inhibition der Protease Trypsin wurde als Substrat das Peptid CBZ-R-pNA (1 mg/ml) sowie TPCK-behandeltes Trypsin (0,1 mg/ml) verwendet und der Test im übrigen wie beschrieben durchgeführt.

Als Kontrolle wurde je ein Ansatz ohne BBI oder Peptide (0) durchgeführt, dessen Meßwert mit 100 % Chymotrypsininaktivität (% CH) bzw. 100 % Trypsinaktivität (% T) gleichgesetzt wurde.

In Fig. 4 ist die Inhibition von Chymotrypsin und in Fig. 5 die Inhibition von Trypsin gezeigt.

Die mit O bezeichnete Kontrolle zeigt die Chymotrypsin- bzw. Trypsin-Aktivität in Abwesenheit von BBI bzw. einem erfindungsgemäßen Peptid.

Wie aus Fig. 4 ersichtlich ist, inhibiert ein Zusatz von 0,05 mM und 0,1 mM Gesamt-BBI (BBI) die Chymotrypsin-Aktivität um 80 bzw. 90 %. Im Vergleich dazu inhibiert der Zusatz von 0,1 mM des Peptids P1, P2, P3 oder P4 die Chymotrypsin-Aktivität nicht oder nur um wenige Prozent.

Bei diesen Untersuchungen handelt es sich natürlich nur um eine sehr grobe Abschätzung, so daß eine weitere Messung der Hemmung von Chymotrypsin durch BBI bzw. das Peptid P2 durchgeführt wurde. Hierzu wurde die Restaktivität durch Hydrolyse des Substrates AAPF-pNA (Alanin-Alanin-Prolin-Phenylalanin-Nitroanilid) quantifiziert. Dabei zeigte sich, daß die Antichymotrypsin-Aktivität des Peptides P2 mindestens um einen Faktor von 500 000 geringer ist als die inhibitorische Aktivität von BBI, da eine etwa gleiche Hydrolysekinetik mit BBI in der Konzentration von 10⁻⁸ M und des Peptides P2 in der Konzentration von 2 x 10⁻³ M beobachtbar ist. Bei diesem Versuch wurde eine deutlich geringere Chymotrypsin-Aktivität (0,28 mU) eingesetzt als bei dem Versuch, der zu den Ergebnissen gemäß Fig. 4 führte, so daß der neue Versuch deutlich empfindlichere Messungen ermöglichte.

Bei einem Aktivitätsunterschied von mehr als fünf Größenordnungen kann nicht mehr von einer inhibitorischen Aktivität des Peptides P2 gesprochen werden.

Wie aus den in Fig. 5 gezeigten Ergebnissen hervorgeht, gilt das gleiche für die Trypsin-inhibierende Aktivität. Während der Gesamt-BBI in einer Konzentration von 0,05 mM oder 0,1 mM die Aktivität von Trypsin um über 90 % inhibiert, zeigt der Zusatz von 0,1 mM jedes der Peptide P1, P2, P3 oder P4 keine wesentliche Auswirkung auf die Aktivität von Trypsin; d.h. die Auswirkung ist in der gewählten Auftragung nicht sichtbar zu machen.

Dieses Beispiel zeigt, daß keines der erfindungsgemäß verwendeten Peptide eine wesentliche inhibierende Wirkung auf die Proteasen Chymotrypsin oder Trypsin ausübt.

Die erfindungsgemäßen verwendeten Peptide haben daher nicht den unerwünschten Nebeneffekt, gleichzeitig auch die Verdauungsenzyme Chymotrypsin und Trypsin zu blockieren. Wie in Versuchen mit Ratten gezeigt wurde, führt die Blockierung dieser im Pankreas (der Bauchspeicheldrüse) hergestellten Proteasen durch Protease-Inhibitoren zu schweren Schädigungen des Pankreas.

## Patentansprüche

1. Verwendung zumindest eines Peptides zur Herstellung eines Mittels zum Schutz von Zellen im Zusammenhang mit einer *in vivo*-Behandlung mit chemischen Agenzien, wobei dieses Peptid ein Fragment des Bowman-Birk-Protease-Inhibitors (BBI) umfaßt.

2. Verwendung zumindest eines Peptides zur Herstellung eines Mittels zum Schutz von Zellen im Zusammenhang mit einer *in vitro*-Behandlung mit chemischen Agenzien, wobei dieses Peptid ein Fragment des Bowman-Birk-Protease-Inhibitors (BBI) umfaßt.

3. Verwendung nach Anspruch 1 oder 2, **dadurch gekennzeichnet, daß** das Peptid keine oder zumindest keine wesentliche Protease-inhibierende Wirkung gegen Trypsin und Chymotrypsin aufweist.

4. Verwendung nach einem der Ansprüche 1 bis 3, **dadurch gekennzeichnet, daß** das Peptid zumindest zwei Cysteinreste aufweist, die in reduzierter Form vorliegen.

5. Verwendung nach einem der Ansprüche 1 bis 4, **dadurch gekennzeichnet, daß** das Peptid weniger als 20, vorzugsweise weniger als 10 Aminosäuren aufweist.

6. Verwendung nach Anspruch 5, **dadurch gekennzeichnet, daß** das Peptid die Sequenz SEQ ID-Nr.: 1: Cys Ala Leu Ser Tyr Pro Ala Gln Cys aufweist.

7. Verwendung nach Anspruch 5, **dadurch gekennzeichnet, daß** das Peptid die Sequenz SEQ ID-Nr.: 2: Cys Ala Leu Val Tyr Pro Ala Gln Cys aufweist.

8. Verwendung nach Anspruch 5, **dadurch gekennzeichnet, daß** das Peptid die Sequenz SEQ ID-Nr.: 3: Ala Leu Ser Tyr Pro Ala Gln aufweist.

9. Verwendung nach einem der Ansprüche 1 bis 8, **dadurch gekennzeichnet, daß** zumindest ein Aminosäurerest des Peptides in alkylierter Form vorliegt.

10. Verwendung nach Anspruch 6 oder 7, **dadurch gekennzeichnet, daß** die terminalen Cysteinreste des Peptides kovalent miteinander verbunden sind.

11. Verwendung nach einem der Ansprüche 1 bis 10, **dadurch gekennzeichnet, daß** zumindest eine Aminosäure des Peptides eine Schutzgruppe aufweist.

12. Verwendung nach Anspruch 11, **dadurch gekennzeichnet, daß** das Peptid an seiner N-terminalen Aminosäure eine Acetylgruppe aufweist.

13. Verwendung nach Anspruch 11 oder 12, **dadurch gekennzeichnet, daß** das Peptid an seiner C-terminalen Aminosäure eine Amidgruppe aufweist.

14. Verwendung zumindest einer Nukleinsäure zur Herstellung eines Mittels zum Schutz von gesunden Zellen im Zusammenhang mit einer Behandlung mit chemischen Agenzien, **dadurch gekennzeichnet, daß** diese Nukleinsäure einen Sequenzabschnitt umfaßt, der für mindestens ein Peptid aus der Verwendung nach einem der Ansprüche 1 bis 8 codiert.

15. Verwendung nach Anspruch 14, **dadurch gekennzeichnet, daß** die Nukleinsäure Sequenzen aufweist, die zur Expression der dem Peptid entsprechenden Nukleinsäuresequenz notwendig sind.

16. Verwendung nach Anspruch 14 oder 15, **dadurch gekennzeichnet, daß** die Nukleinsäure in einem Vektor enthalten ist, der geeignet ist, die Expression der dem Peptid entsprechenden Nukleinsäuresequenz in einer Zelle zu ermöglichen.

17. Verwendung einer nach einem der Ansprüche 14 bis 16 verwendeten Nukleinsäure im Rahmen einer Gentherapie.

18. Verwendung einer nach einem der Ansprüche 14 bis 16 verwendeten Nukleinsäure zur Transformation oder Transfektion von Zellen in vitro.

19. Verwendung nach einem der Ansprüche 1 bis 18 zum Schutz von Zellen im Zusammenhang mit einer Behandlung mit Zytostatika, insbesondere Alkylanzien, wie Cisplatin oder Nitroso-Harnstoff-/-Guanidin-Verbindungen, Antimetabolite, wie Fluorouracil und/oder Antibiotika, wie Bleomycin.

20. Verwendung nach einem der Ansprüche 1 bis 18 zum Schutz von Zellen im Zusammenhang mit einer Behandlung mit Virostatika, insbesondere Nukleosidanaloga, wie Azidothymidin, Aciclovir oder Idoxuridin.

21. Verwendung nach einem der Ansprüche 1 bis 18 zum Schutz von Zellen im Zusammenhang mit einer Behandlung mit immunsupprimierenden Mitteln, wie bspw. Azathioprin.

22. Verwendung nach einem der Ansprüche 1 bis 21, **dadurch gekennzeichnet, daß** die Peptide und/oder Nukleinsäuren an einen Hilfsstoff gekoppelt oder an diesen gebunden sind.

23. Verwendung nach einem der Ansprüche 1 bis 22, **dadurch gekennzeichnet, daß** die Peptide und/oder Nukleinsäuren in einer Zellen gegenüber Zytostatika, immunsupprimierenden Mitteln und/oder Virostatika schützenden wirksamen Menge Bestandteil einer pharmazeutischen Zusammensetzung sind.

## Claims

1. Use of at least one peptide for preparing a means for protecting cells in connection with an *in-vivo* treatment with chemical agents, said peptide comprising a fragment of the Bowman-Birk protease inhibitor (BBI).

2. Use of at least one peptide for preparing a means for protecting cells in connection with an *in-vitro* treatment with chemical agents, said peptide comprising a fragment of the Bowman-Birk protease inhibitor (BBI).

3. Use according to claim 1 or 2, **characterized in that** said peptide exhibits no, or at least no significant, protease-inhibiting effect against trypsin and chymotrypsin.

4. Use according to any of claims 1 to 3, **characterized in that** said peptide comprises at least two cysteine residues, which are present in reduced form.

5. Use according to any of claims 1 to 4, **characterized in that** said peptide comprises less than 20, preferably less than 10, amino acids.

6. Use according to claim 5, **characterized in that** said peptide comprises the sequence SEQ ID No.: 1: Cys Ala Leu Ser Tyr Pro Ala Gln Cys.

7. Use according to claim 5, **characterized in that** said peptide comprises the sequence SEQ ID No.: 2: Cys Ala Leu Val Tyr Pro Ala Gln Cys.

8. Use according to claim 5, **characterized in that** said peptide comprises the sequence SEQ ID No.: 3: Ala Leu Ser Tyr Pro Ala Gln.

9. Use according to any of claims 1 to 8, **characterized in that** at least one amino acid residue of the peptide is present in alkylated form.

10. Use according to claim 6 or 7, **characterized in that** the terminal cysteine residues of the peptide are covalently linked to each other.

11. Use according to any of claims 1 to 10, **characterized in that** at least one amino acid in the peptide comprises a protecting group.

12. Use according to claim 11, **characterized in that** the peptide comprises an acetyl group at its N-terminal amino acid.

13. Use according to claim 11 or 12, **characterized in that** the peptide comprises an amide group at its C-terminal amino acid.

14. Use of at least one nucleic acid for preparing a means for protecting healthy cells in connection with a treatment with chemical agents, **characterized in that** this nucleic acid comprises a sequence segment which encodes at least one peptide from the use according to any of claims 1 to 8.

15. Use according to claim 14, **characterized in that** said nucleic acid comprises sequences which are necessary for expressing the nucleic acid sequence which corresponds to the peptide.

16. Use according to claim 14 or 15, **characterized in that** said nucleic acid is contained in a vector which is suitable for enabling the nucleic acid sequence which corresponds to the peptide to be expressed in a cell.

17. Use of a nucleic acid which is used in accordance with any of claims 14 to 16 within the context of a gene therapy.

18. Use of a nucleic acid which is used in accordance with any of claims 14 to 16 for transforming or transfecting cells *in vitro*.

19. Use according to any of claims 1 to 18 for protecting cells in connection with a treatment with cytostatic agents, in particular alkylating agents, such as cisplatin or nitroso-urea/-guanidine compounds, antimetabolites, such as fluorouracil, and/or antibiotics, such as bleomycin.

20. Use according to any of claims 1 to 18 for protecting cells in connection with a treatment with virostatic agents, in particular nucleoside analogues, such as azidothymidine, acyclovir or idoxuridine.

21. Use according to any of claims 1 to 18 for protecting cells in connection with a treatment with immunosuppressing agents, such as azathioprine.

22. Use according to any of claims 1 to 21, **characterized in that** said peptides and/or nucleic acids are coupled or bound to an auxiliary substance.

23. Use according to any of claims 1 to 22, **characterized in that** said peptides and/or nucleic acids are, in an effective quantity which protects cells against cytostatic agents, immunosuppressing agents and/or virostatic agents, constituents of a pharmaceutical composition.

## Revendications

1. Utilisation d'au moins un peptide pour la préparation d'un agent destiné à la protection des cellules en rapport avec un traitement *in vivo* avec des agents chimiques, ce peptide comprenant un fragment de l'inhibiteur de protéase de Bowman - Birk (BBI).

2. Utilisation d'au moins un peptide pour la préparation d'un agent destiné à la protection des cellules en rapport avec un traitement *in vitro* avec des agents chimiques, ce peptide comprenant un fragment de l'inhibiteur de protéase de Bowman - Birk (BBI).

3. Utilisation selon la revendication 1 ou 2, **caractérisée en ce que** le peptide ne présente aucune action ou du moins aucune action essentielle inhibitrice de protéase contre la trypsine et la chymotrypsine.

4. Utilisation selon l'une quelconque des revendications 1 à 3, **caractérisée en ce que** le peptide comprend au moins deux résidus de cystéine qui se présentent sous forme réduite.

5. Utilisation selon l'une quelconque des revendications 1 à 4, **caractérisée en ce que** le peptide présente moins de 20, de préférence moins de 10 acides aminés.

6. Utilisation selon la revendication 5, **caractérisée en ce que** le peptide comprend la séquence SEQ ID n°1 : Cys Ala Leu Ser Tyr Pro Ala Gln Cys.

7. Utilisation selon la revendication 5, **caractérisée en ce que** le peptide comprend la séquence SEQ ID n°2 : Cys Ala Leu Val Tyr Pro Ala Gln Cys.

8. Utilisation selon la revendication 5, **caractérisée en ce que** le peptide comprend la séquence SEQ ID n°3 : Ala Leu Ser Tyr Pro Ala Gln.

9. Utilisation selon l'une quelconque des revendications 1 à 8, **caractérisée en ce qu'**au moins un résidu d'acide aminé du peptide se présente sous forme alkylée.

10. Utilisation selon la revendication 6 ou 7, **caractérisée en ce que** les résidus de cystéine terminaux du peptide sont liés entre eux de manière covalente.

11. Utilisation selon l'une quelconque des revendications 1 à 10, **caractérisée en ce qu'**au moins un acide aminé du peptide comprend un groupe protecteur.

12. Utilisation selon la revendication 11, **caractérisée en ce que** le peptide comprend sur son acide aminé N-terminal un groupe acétyle.

13. Utilisation selon la revendication 11 ou 12, **caractérisée en ce que** le peptide comprend sur son acide aminé C-terminal un groupe amide.

14. Utilisation d'au moins un acide nucléique pour la préparation d'un agent de protection des cellules saines en rapport avec un traitement avec des traitements chimiques, **caractérisée en ce que** cet acide nucléique comprend une section de séquence, qui code au moins un peptide provenant de l'utilisation selon l'une quelconque des revendications 1 à 8.

15. Utilisation selon la revendication 14, **caractérisée en ce que** l'acide nucléique comprend des séquences nécessaires à l'expression de la séquence d'acides nucléiques correspondant au peptide.

16. Utilisation selon la revendication 14 ou 15, **caractérisée en ce que** l'acide nucléique est contenu dans un vecteur approprié à permettre l'expression de la séquence d'acides nucléiques correspondant au peptide dans une cellule.

17. Utilisation d'un acide nucléique utilisé selon l'une quelconque des revendications 14 à 16 dans le cadre d'une thérapie génique.

18. Utilisation d'un acide nucléique utilisé selon l'une quelconque des revendications 14 à 16 pour la transformation ou la transfection de cellules in vitro.

19. Utilisation selon l'une quelconque des revendications 1 à 18 pour la protection des cellules en rapport avec le traitement avec des cytostatiques, en particulier les alkylants, comme la cisplatine ou les composés nitrosourée/guanidine, des antimétabolites comme le fluorouracil et/ou des antibiotiques comme bléomycine.

20. Utilisation selon l'une quelconque des revendications 1 à 18 pour la protection des cellules en rapport avec un traitement avec des virostatiques, en particulier des analogues de nucléosides, comme l'azidothymidine, l'aciclovir ou l'idoxuridine.

21. Utilisation selon l'une quelconque des revendications 1 à 18 pour la protection des cellules en rapport avec un traitement avec des agents immunosuppresseurs comme par exemple l'azathioprine.

22. Utilisation selon l'une quelconque des revendications 1 à 21, **caractérisée en ce que** les peptides et/ou les acides nucléiques sont couplés à un auxiliaire ou sont liés à celui-ci.

23. Utilisation selon l'une quelconque des revendications 1 à 22, **caractérisée en ce que** les peptides et/ou les acides nucléiques font partie des composants d'une composition pharmaceutique en une quantité efficace, protégeant les cellules contre les cytostatiques, agents immunosuppresseurs et/ou virostatiques.


### SEQUENZPROTOKOLL

<110> Eberhard-Karls-Universitaet Tuebingen, Uni-Klinik
<120> Schutz von Zellen im Zusammenhang mit einer Behandlung mit chemischen Agenzien
<130> 5402P165
<140>
<141>
<160> 3
<170> PatentIn Ver. 2.1

<210> 1
<211> 9
<212> PRT
<213> Künstliche Sequenz
<220>
<223> Beschreibung der künstlichen Sequenz: Fragment aus dem Bowman-Birk-Protease-Inhibitor der Sojabohne

<400> 1
   Cys Ala Leu Ser Tyr Pro Ala Gln Cys
     1               5

<210> 2
<211> 9
<212> PRT
<213> Künstliche Sequenz
<220>
<223> Beschreibung der künstlichen Sequenz: In einem Fragment aus dem Bowman-Birk-Protease-Inhibitor aus der Sojabohne wurde ein Ser durch ein Val ersetzt.

<400> 2
   Cys Ala Leu Val Tyr Pro Ala Gln Cys
   1 5

<210> 3
<211> 7
<212> PRT
<213> Künstliche Sequenz
<220>
<223> Beschreibung der künstlichen Sequenz: Fragment aus dem Bowman-Birk-Protease-Inhibitor der Sojabohne

<400> 3
   Ala Leu Ser Tyr Pro Ala Gln
   1 5